# FASCICULE DE BREVET EUROPEEN

(11) **EP 0 623 385 B1**
(45) Date de publication et mention de la délivrance du brevet: **29.09.1999**
(21) Numéro de dépôt: 94400888.7
(22) Date de dépôt: 25.04.1994
(51) Int. Cl.: B01J 23/62, B01J 37/02, C10G 35/09, B01J 23/56, B01J 23/76

(54) **Procédé de préparation de catalyseurs applicables à la déshydrogénation**
Verfahren zur Herstellung von Dehydrierungskatalysatoren
Process for the preparation of dehydrogenation catalysts

(30) Priorité: 06.05.1993 FR 9305555
(43) Date de publication de la demande: 09.11.1994
(73) Titulaire: INSTITUT FRANCAIS DU PETROLE, 92502 Rueil-Malmaison (FR)
(72) Inventeur: Le Peltier, Fabienne, F-92500 Rueil Malmaison (FR); Robert, Sylvie, F-92500 Rueil Malmaison (FR); Boitiaux, Jean-Paul, F-78300 Poissy (FR); Didillon, Blaise, F-92500 Rueil Malmaison (FR); Clause, Olivier, F-78400 Chatou (FR)

(56) Documents cités:
- FR-A- 2 539 647
- FR-A- 2 545 380
- FR-A- 2 594 711
- US-A- 3 531 543
- US-A- 4 888 104

## Description

La présente invention concerne un nouveau procédé de préparation d'un catalyseur à base d'au moins un métal du groupe VIII de la classification périodique des éléments modifié par ajout d'au moins un métal additionnel qu'il est nécessaire de mettre en intéraction avec le métal de base pour obtenir un nouveau catalyseur plus performant, et éventuellement au moins un autre métal choisi dans le groupe constitué par les métaux alcalins et/ou un métalloide tel que le soufre ou tout autre élément chimique tel qu'un halogène ou un composé halogéné.

Les brevets et publications démontrant que l'addition de promoteurs à un métal de base améliore la qualité des catalyseurs sont fort nombreux. Ces éléments sont ajoutés sous différentes formes tels que sels ou composés organométalliques. On obtient généralement des catalyseurs plus actifs ou plus sélectifs, partois plus stable qu'avec le métal de base.

La façon dont ces modificateurs sont apportés n'est pas indifférente car il est nécessaire que les divers éléments du catalyseur soient intimement associés pour obtenir un effet notable. La présente invention concerne la modification d'un catalyseur appelé ci-après "précatalyseur" de départ par ajout dans des conditions bien contrôlées des éléments modificateurs directement dans le réacteur où l'utilisation du catalyseur doit être faite. Cette façon de faire présente de nombreux avantages pratiques mais on a pu constater que, de manière surprenante, qu'un tel mode de fabrication permettait l'obtention de catalyseurs bien plus performants que ceux obtenus par préparation hors site.

Ces catalyseurs renferment au moins un support, au moins un métal de la famille du groupe VIII, un métal additionnel (dénommé ci-après métal M) choisi parmi le germanium, l'étain, le plomb, le fer, le titane et le chrome. Le catalyseur contient aussi éventuellement et de manière préférée au moins un métal alcalin ou alcalino-terreux et éventuellement également comme indiqué ci-dessus un élément choisi parmi les métalloides (soufre par exemple) et les halogènes ou les composés halogénés.

Ils s'utilisent en particulier dans un procédé catalytique de déshydrogénation d'une charge d'hydrocarbures comportant principalement des paraffines comprenant de 2 à 22 atomes de carbone par molécule, c'est-à-dire des paraffines C₂-C₂₂. La réaction de déshydrogénation s'effectue en général à une pression comprise entre 0,2 et 20 bar absolus (1 bar = 0,1 MPa) sous une pression préférée de 1 à 10 bars absolus, et à une température comprise entre 400 et 800 °C en fonction de la nature de la charge. La température est avantageusement comprise entre 560 et 700 °C pour une charge comprenant principalement du propane, entre 450 et 600 °C pour une charge comprenant pricipalement de l'isobutane et entre 400 et 550 °C pour une charge comprenant principalement de l'isopentane. La température est avanta-geusement comprise entre 400 et 550 °C pour une charge contenant principalement des paraffines comprenant de 9 à 22 atomes de carbone par molécule. La charge peut aussi contenir des hydrocarbures insaturés comportant de 2 à 22 atomes de carbone par molécule. Il peut être avantageux d'utiliser de l'hydrogène comme diluant. Le rapport molaire hydrogène/hydrocarbure est généralement compris entre 0 et 20 et de préférence entre 0 et 6. Les vitesses spatiales volumiques (par rapport à la charge liquide) préconisées sont habituellement de 0,5 à 100 h⁻¹ et préférentiellement de 1,5 à 50 h⁻¹.

Les formulations des catalyseurs de déshydrogénation ont fait l'objet d'un très grand nombre d'études. Les catalyseurs métalliques supportés ont été décrits notamment dans les brevets US-A-3 531 543 et US-A-3 998 900. Ils contiennent une phase métallique à base de platine, modifiée par un métal additionnel M tel que l'étain, supportés sur des oxydes inorganiques réfractaires telle que l'alumine. L'introduction du métal M est avantageusement effectuée à l'aide d'un composé organométallique dudit métal M. Cette méthode d'introduction du métal M a déjà été décrite dans le brevet US-A-3 531 543.

On a maintenant découvert, et ceci fait l'objet de la présente invention, une nouvelle méthode d'introduction du métal additionnel M caractérisée en ce que le dit métal additionnel M est introduit par mise en contact sous atmosphère inerte (par exemple azote) ou réductrice (par exemple hydrogène) d'au moins un composé organique du métal additionnel M pur ou éventuellement dans au moins une solution hydrocarbonée, avec le précatalyseur chargé dans le réacteur où seront injectées la ou les charges réactionnelles, par exemple les hydrocarbures qui subiront la transformation de déshydrogénation en présence du catalyseur ainsi préparé. Le précatalyseur est, dans la présente invention un catalyseur renfermant au moins un support, au moins un métal du groupe VIII de la classification périodique des éléments, évetuellement au moins un métal alcalin ou alcalino-terreux, éventuellement au moins un métalloide ou un halogène ou un composé halogéné. Ce catalyseur ne renferme pas ledit métal additionnel M.

L'addition de ce métal additionnel M est réalisée in-situ en phase liquide ou en phase gazeuse c'est à dire dans le réacteur industriel (zone de réaction) ou dans un préréacteur industriel (prézone de réaction) directement relié au dit réacteur et où le précatalyseur, précurseur du catalyseur fini est chargé. Cette opération de fixation du métal additionnel M peut être réalisée entre 20 et 500 °C.

On a ainsi découvert qu'en opérant en présence de catalyseurs préparés selon l'invention, ces catalyseurs possédaient une activité et une durée de vie accrues et une meilleure régénérabilité, par rapport aux catalyseurs de l'art antérieur, préparés selon les techniques de l'art antérieur.

Le support du catalyseur selon l'invention comporte au moins un oxyde réfractaire qui est généralement choisi parmi les oxydes des métaux des groupes IIA, IIA ou IVA de la classification périodique des éléments tels que par exemple les oxydes de magnésium, d'aluminium, de silicium pris seuls ou en mélange entre eux ou en mélange avec d'autres oxydes d'éléments de la classification périodique. Le support préféré est l'alumine, dont la surface spécifique est avantageusement comprise entre 50 et 400 m² par gramme, de préférence entre 100 et 400 m² par gramme.

Le métal noble est choisi parmi les métaux nobles du groupe VIII tels que le platine, le palladium, le nickel, le ruthénium, de préférence le platine.

Le métal additionnel M est choisi parmi le germanium, l'étain, le plomb, le fer, le titane et le chrome.

Le catalyseur contient éventuellement et de manière préférée au moins un métal alcalin ou alcalino-terreux. Dans le cas où le catalyseur est utilisé pour la déshydrogénation d'une charge d'hydrocarbures comprenant essentiellement des oléfines C₂-C₅, le métal alcalin ou alcalino-terreux préféré est le potassium. Dans le cas où le catalyseur est utilisé pour la déshydrogénation d'une charge d'hydrocarbures comprenant principalement des paraffines C₉-C₂₂, et éventuellement des paraffines C₉-C₁₅ ou encore C₇-C₈, le métal alcalin ou alcalino-terreux préféré est le lithium.

Le catalyseur contient en outre éventuellement du soufre. Le catalyseur contient en outre éventuellement au moins un halogène, c'est à dire au moins un élément du groupe VIIA tel que le chlore.

Le catalyseur selon l'invention renferme de préférence en poids par rapport au support :
(a) de 0,01 à 2 % d'au moins un métal noble de la famille du groupe VII
(b) de 0,01 à 3 % d'au moins un élément additionnel M
(c) de 0,1 à 3 % d'au moins un métal alcalin ou alcalino-terreux lorsque le catalyseur renferme un tel métal.

Une des formules catalytiques préférées selon l'invention contient de 0,1 à 1 % poids de platine, de 0,01 à 1 % en poids du métal additionnel M et de 0,1 à 1,5 % en poids de potassium.

Le catalyseur peut contenir de 0,005 à 1 % poids de soufre. Le catalyseur peut aussi contenir de 0,005 à 2,5 % poids d'un halogène tel que le chlore.

La préparation du précatalyseur, précurseur du catalyseur fini, se fait selon toute technique connue de l'homme du métier.

Dans une technique préférée de préparation du catalyseur selon l'invention, le précatalyseur est préparé à partir d'un support préformé selon les méthodes classiques consistant à imprégner le support au moyen de solutions de composés des éléments que l'on désire introduire. On utilise soit une solution commune des métaux présents dans le catalyseur soit des solutions distinctes dans un ordre quelconque. Quand on utilise plusieurs solutions, on peut procéder à des séchages et/ou des calcinations intermédiaires. On termine habituellement par une calcination par exemple entre 300 et 650 °C; de préférence en présence d'oxygène libre, par exemple en effectuant un balayage d'air.

Le métal alcalin ou alcalino-terreux, lorsque le catalyseur contient ce type de métal, peut être introduit dans le support au moyen d'une solution aqueuse contenant des sels décomposables dudit métal sous forme de nitrate, de carbonate ou d'acétate, comme par exemple le carbonate de potassium.

L'introduction du métal du groupe VIII est effectuée de préférence par imprégnation du support par une solution aqueuse d'un composé halogéné. Le platine est préférentiellement introduit sous forme d'acide chloroplatinique. Après l'introduction du métal du groupe VIII, le produit obtenu est calciné après un éventuel séchage ; la calcination est effectuée de préférence à une température comprise entre 300 et 680 °C en présence d'un composé organique halogéné (oxychloration). Les composés organiques halogénés sont choisis par exemple dans le groupe formé par le tétrachlorure de carbone, le chloroforme, le dichlorométhane et le dichloropropane.

En vue de procéder à l'introduction du métal M dans le précatalyseur, on commence par charger le précatalyseur dans le réacteur industriel ou dans le préréacteur industriel. Avant de procéder à ce chargement, ou dans le réacteur industriel ou dans le préréacteur industriel, le précatalyseur est éventuellement séché et est soumis à une calcination sous atmosphère oxydante entre 300 et 650 °C. Selon l'invention, le précatalyseur est ensuite soumis à un traitement d'activation sous atmosphère réductrice (hydrogène) ou neutre (azote). La solution préférée est un traitement d'activation sous hydrogène à haute température par exemple entre 300 et 600 °C. Cette réduction peut consister par exemple en une montée lente de la température sous courant d'hydrogène jusqu'à la température maximale de réduction, comprise par exemple entre 300 et 600 °C suivie d'un maintien sous hydrogène pendant 1 à 6 heure à cette température.

Après avoir ajusté la température à valeur désirée, comprise entre 20 et 500 °C, de préférence sous débit d'hydrogène, on procède ensuite à l'introduction du métal M. Avant mise en contact avec la charge à traiter, par exemple les réactifs des réactions de déshydrogénation d'hydrocarbures, le solvant d'imprégnation est éliminé si nécessaire, par exemple par soutirage et strippage, et l'on procède à un traitement thermique sous débit gazeux, d'hydrogène de préférence, à haute température, comprise entre 300 et 600 °C , pendant plusieurs heures.

Le métal additionnel M est introduit dans le précatalyseur sous la forme d'au moins un composé organométallique ou alcoolate choisi dans le groupe formé par les complexes, en particulier les complexes carbonyles, polycétoniques des métaux M, et les hydrocarbylmétaux du métal M tels que les alkyles, les cycloalkyles, les aryles, les alkylaryles métaux et les arylalkyles métaux.

L'introduction du métal M est avantageusement effectuée à l'aide d'une solution dans un solvant organique du composé alcoolate ou organométallique dudit métal M. On peut également employer des composés organo-halogénés des métaux M. Comme composés de métaux M on citera en particulier, l'hexacarbonyle de fer, l'isopropylate de titane, le dichloro-dicyclopentadienyle de titane, le tétrabutylétain, le tétraméthylétain, le tétrapropylgermanium, le diphénylétain, le tétraéthylplomb.

Le solvant d'imprégnation est choisi dans le groupe constitué par les solvants organiques oxygénés contenant de 2 à 8 atomes de carbone par molécule, et les hydrocarbures paraffiniques, naphténiques ou aromatiques contenant essentiellement de 6 à 15 atomes de carbone par molécule et les composés organiques halogénés contenant de 1 à 15 atomes de carbone par molécule. On peut citer l'éthanol, le tétrahydrofuranne, le n-heptane, le méthyl cyclohexane, le toluène et le chloroforme. On peut utiliser ces solvants pris seuls ou en mélange entre eux.

Une méthode préférée de préparation de catalyseurs selon l'invention consiste à opérer comme suit :
(a) on imprègne un support, contenant éventuellement un composé alcalin ou alcalino-terreux, à l'aide d'une solution aqueuse contenant au moins un métal du groupe VIII. On obtient ainsi une masse catalytique appelée précédemment "précatalyseur".
(b) on sèche éventuellement la masse catalytique (ou précatalyseur) obtenue,
(c) on calcine éventuellement la masse catalytique obtenue, puis conformément à l'invention,
(d) on charge la masse catalytique obtenue dans un réacteur industriel ou dans un préréacteur industriel
(e) on réduit la masse catalytique calcinée chargée dans le réacteur
(f) on met en contact la masse catalytique réduite chargée dans le réacteur avec le composé organique du métal addtionnel M pur ou solubilisé dans un solvant hydrocarboné
(g) si nécessaire on élimine le solvant
(h) on réduit la masse catalytique contenant le platine ou le métal noble de la famille du platine et le métal additionnel M.

Les exemples qui suivent illustrent l'invention sans toutefois en limiter la portée.

### EXEMPLE 1

On prépare trois catalyseurs A, B et C renfermant 0,6 % en poids de platine, 0,45 % en poids d'étain, 1 % en poids de potassium et 1,5 % en poids de chlore. Le support est une alumine de surface spécifique de 220 m² par gramme et de volume poreux de 0,60 cm³ par gramme.

### Préparation du catalyseur A (comparatif)

On prépare un catalyseur A selon les techniques de l'art antérieur. Le support est une alumine de surface spécifique de 220 m² par gramme et de volume poreux de 0,60 cm³ par gramme.

A 100 g de support d'alumine on ajoute 500 cm³ d'une solution aqueuse d'acide chlorhydrique. On laisse en contact trois heures, on essore, on sèche 1 heure à 120 °C. Sur le produit séché contenant du chlore, on procède alors à l'imprégnation du platine et de l'étain en ajoutant au solide 150 cm³ d'une solution d'acide hexachloroplatine et de chlorure stannique. La concentration en platine de cette solution est égale à 4,05 g par litre et la concentration en étain est de 3,04 g par litre. On laisse en contact 6 heures, on séche 1 heure à 120 °C puis on calcine 2 heures à 530 °C. Sur le produit calciné on procède à l'introduction du potassium par ajout de 60 cm³ d'une solution aqueuse contenant 1,7 g de carbonate de potassium, ensuite on l'échantillon est séché à 120 °C puis calciné pendant 2 heures à 530 °C.

### Préparation du catalyseur B (comparatif)

Le catalyseur B est préparé à partir de 80 g de support alumine. Le solide est d'abord calciné à 530 °C pendant 2 heures sous un débit d'air de 80 litres par heure. On ajoute 48 cm³ d'une solution aqueuse contenant 1,4 g de carbonate de potassium puis l'échantillon est calciné pendant 2 heures à 530 °C. On procède ensuite à l'introduction du platine et du chlore par ajout de 48 cm³ d'une solution aqueuse d'acide hexachloroplatinique et d'acide chlorydrique contenant en tout 0,48 g de platine et 1,2 g de chlore. On laisse 4 heures en contact, on sèche 1 heure à 120 °C puis on calcine pendant 2 heures à 530 °C. On réduit ensuite le catalyseur 2 heures sous un débit d'hydrogène de 80 litres par heure à 450 °C.

Ensuite sur 15 g du produit réduit contenant le potassium et le platine, on procède alors à l'imprégnation de l'étain dans un ballon réactionnel, en ajoutant 45 cm³ d'une solution de n-heptane contenant 0,4 g de tétrabutylétain. On laisse en contact 8 heures à la température ambiante sous un débit d'hydrogène de 85 êtres par heure. Le solide obtenu est essoré puis séché à 120 °C et ensuite calciné à 530 °C pendant 2 heures.

### Préparation du catalyseur C (selon l'invention)

3,5 g de catalyseur calciné contenant le potassium et le platine sont préparés dans les mêmes conditions que celles du catalyseur B. Le catalyseur est ensuite chargé dans un réacteur tubulaire isotherme fonctionnant en flux descendant à la pression atmosphérique.

Le catalyseur est ensuite réduit 2 heures dans le réacteur sous 20 litres par heure d'hydrogène à 530 °C. La température est alors diminuée à la température ambiante tout en conservant le débit d'hydrogène. On fait alors circuler une solution de n-heptane contenant 0.2 g de tétrabutylétain. La circulation du solvant est maintenue pendant 8 heures à la température ambiante toujours sous débit d'hydrogène. Le solvant est alors éliminé par soutirage et l'on procède à une réduction du catalyseur sous un débit d'hydrogène de 20 litres par heure pendant 2 heures à 530 °C. Ce catalyseur appelé catalyseur C est directement utilisable pour la réaction catalytique envisagée.

### EXEMPLE 2

Les catalyseurs A, B et C sont soumis à un test de déshydrogénation d'une charge d'isobutane pur (99,9 % d'isobutane et 0,1 % de n-butane) réalisé dans le réacteur tubulaire isotherme utilisé pour la préparation du catalyseur C. Pour les tests des catalyseur A et B, 3,5 g de ces catalyseurs ont été préalablement chargés dans le récateur puis réduits à 530 °C sous 20 litres par heure d'hydrogène pendant 2 heures.

Ensuite on injecte 20 litres par heure d'isobutane, ce qui correspond à un rapport molaire hydrogène sur hydrocarbure de 1 et à une vitesse spatiale massique de 14 h⁻¹, puis on augmente la température à 580 °C. L'analyse des effluents gazeux se fait en ligne par chromatographie en phase gazeuse.

Les résultats obtenus dans ces conditions, exprimés en % poids, sont rapportés dans le tableau 1.

**Tableau 1**

| Catalyseur | Nombre de cycles | conversion en iC₄ (%pds) | sélectivité en iC₄= (%pds) | rendement en iC₄= (%pds) |
|---|---|---|---|---|
| A | 1 | 38,0 | 85,0 | 32,3 |
| | 4 | 33,6 | 88,3 | 29,7 |
| | 6 | 32,4 | 89,5 | 29,0 |
| B | 1 | 47,5 | 87,0 | 41,3 |
| | 4 | 43,4 | 89,4 | 38,8 |
| | 6 | 42,8 | 89,8 | 38,4 |
| C | 1 | 53,8 | 86,8 | 46,7 |
| | 4 | 50,7 | 89,0 | 45,1 |
| | 6 | 49,0 | 90,0 | 44,1 |

Le catalyseur C préparé selon l'invention, présente de meilleures performances catalytiques que le catalyseur A préparé selon les techniques de l'art antérieur à partir de chlorure d'étain. De plus les propriétés du catalyseur C sont supérieures à celles du catalyseur B, bien que ces deux catalyseurs aient été préparés à partie du même précurseur d'étain, le catalyseur C ayant été préparé directement dans le réacteur tubulaire du test catalytique.

### EXEMPLE 3

Après les tests catalytiques réalisés dans les conditions de l'exemple 2, les catalyseurs A et C sont soumis à des étapes de régénération visant à éliminer les dépôts hydrocarbonés formés au cours de la réaction. Cette étape de régénération est réalisée par calcination à 550 °C du catalyseur sous un débit d'air de 10 litres par heure, directement dans le réacteur tubulaire servant au test catalytique.

Le tableau 2 compare les performances catalytiques des catalyseurs A et C soumis chacun à 4 cycles de réaction-régénération.

**Tableau 2**

| Catalyseur | Nombre de cycles | conversion en iC₄ (%pds) | sélectivité en iC₄= (%pds) | rendement en iC₄= (%pds) |
|---|---|---|---|---|
| A | 1 | 33,5 | 88,0 | 29,5 |
| | 4 | 29,2 | 89,6 | 26,2 |
| | 6 | 28,8 | 90,5 | 26,1 |
| C | 1 | 51,1 | 86,9 | 44,4 |
| | 4 | 49,2 | 89,2 | 43,9 |
| | 6 | 47,8 | 90,2 | 43,1 |

Après 4 cycles de réaction-régénération, le catalyseur C, préparé directement dans le réacteur catalytique, conserve de meilleures performances catalytiques en déshydrogénation de l'isobutane que le catalyseur A ayant ou non subi ces traitements de régénération.

### EXEMPLE 4

On prépare deux catalyseurs D et E à partir du même précurseur monométallique renfermant 0,3 % en poids de platine et 1 % en poids de lithium.

À 150 g de support alumine, on ajoute 90 cm3 d'une solution aqueuse d'acétate de lithium contenant 1,5 g de lithium. On laisse en contact 3 heures, on séche 1 heure à 120 °C puis on calcine 2 heures à 350 °C. Ensuite on procède à l'introduction du platine par ajout de 400 cm³ d'une solution de toluène contenant 0,9 g d'acétylacétonate de platine. On laisse 24 heures en contact puis on séche 1 heure à 120 °C et on calcine 2 heures à 350 °C. Ensuite ce catalyseur est réduit pendant 2 heures à 450 °C.

### Préparation du catalyseur D (comparatif)

Sur 50 g de ce catalyseur platine - lithium, on procède ensuite à l'introduction de l'étain par ajout de 30 cm³ d'une solution de n-heptane contenant 0,75 g de tétrabutylétain. On laisse en contact 3 heures, on sèche 1 heure à 120 °C puis on calcine 2 heures à 350 °C.

### Préparation du catalyseur E (selon l'invention)

On charge 10 g du catalyseur platine-lithium dans un réacteur tubulaire isotherme fonctionnant à la pression atmosphérique. Le catalyseur est ensuite réduit 2 heures à 450 °C dans le réacteur sous 10 l/h d'hydrogène. La température est alors diminuée à la température ambiante tout en conservant le débit d'hydrogène. On fait alors circuler une solution de n-heptane contenant 0.15 g de tétrabutylétain. La circulation du solvant est maintenue pendant 8 heures à la température ambiante toujours sous débit d'hydrogène. Le solvant est alors éliminé par soutirage et l'on procède à une réduction du catalyseur sous un débit d'hydrogène de 10 litres par heure pendant 2 heures à 450 °C. Ce catalyseur E est directement utilisable pour la réaction catalytique envisagée.

### EXEMPLE 5

Les catalyseurs D et E sont soumis à un test de déshydrogénation du n-décane réalisé dans le réacteur tubulaire isotherme utilisé pour la préparation du catalyseur E. Pour le test du catalyseur D, 10 g de ce catalyseur ont été préalablement chargés dans le réacteur puis réduits à 450 °C sous 10 litres par heures d'hydrogène. Les tests catalytiques sont ensuite effectués sous une pression absolue de 3 bars, pour un rapport molaire hydrogène sur hydrocarbure de 10 à une vitesse spatiale massique de 20 h⁻¹, entre 470 et 490 °C.

Les résultats obtenus dans ces conditions, exprimés en % poids, sont rapportés dans le tableau 3.

**Tableau 3**

| Catalyseur | température ( °C) | conversion en nC₁₀ (%pds) | rendement en oléfines (%pds) | rendement en aromatiques (%pds) |
|---|---|---|---|---|
| D | 470 | 10,0 | 8,0 | 0,7 |
| | 490 | 15,5 | 11,9 | 2,2 |
| | 470 | 8,1 | 6,8 | 0,4 |
| E | 470 | 10,5 | 9,2 | 0,4 |
| | 490 | 16,0 | 13,8 | 1,1 |
| | 470 | 10,0 | 9,1 | 0,2 |

Le catalyseur E, préparé selon l'invention directement dans le réacteur catalytique, présente des rendements en oléfines supérieurs et de plus faibles rendements en aromatiques. Ces derniers étant des produits indésirables de la réaction. De plus le catalyseur E présente une meilleure stabilité, comme l'indique le point retour à 470 °C, que le catalyseur D.

### EXEMPLE 6

On prépare trois catalyseurs F, G et H. Les catalyseurs G et H sont préparés à partir d'un précurseur monométallique renfermant 0,3 % en poids de palladium.

A 150 g de support alumine, on ajoute 400 cm³ d'une solution de toluène contenant 1,3 g d'acétylacétonate de palladium. On laisse en contact 24 heures, on séche 1 heure à 120 °C puis on calcine 2 heures à 350 °C. Ensuite ce catalyseur F est réduit pendant 2 heures à 450 °C.

### Préparation du catalyseur G (comparatif)

Sur 50 g de ce catalyseur F, on procède ensuite à l'introduction de l'étain par ajout de 30 cm³ d'une solution de n-heptane contenant 0,62 g de tétrabutylgermane. On laisse en contact 3 heures, on sèche 1 heure à 120 °C puis on calcine 2 heures à 350 °C.

### Préparation du catalyseur H (selon l'invention)

On charge 10 g du catalyseur E dans le réacteur tubulaire isotherme fonctionnant à la pression atmosphérique. Le catalyseur est ensuite réduit 2 heures à 450 °C dans le réacteur sous 10 l/h d'hydrogène. La température est alors diminuée à la température ambiante tout en conservant le débit d'hydrogène. On fait alors circuler une solution de n-heptane contenant 0,12 g de tétrabutylgermane. La circulation du solvant est maintenue pendant 8 heures à la température ambiante toujours sous débit d'hydrogène. Le solvant est alors éliminé et l'on procède à une réduction du catalyseur sous un débit d'hydrogène de 10 litres par heure pendant 2 heures à 450 °C. Ce catalyseur H est directement utilisable pour la réaction catalytique envisagée.

### EXEMPLE 7

Les catalyseurs F, G, H sont soumis à un test de déshydrogénation du n-décane réalisé dans le réacteur tubulaire isotherme utilisé pour la préparation du catalyseur E. Pour les tests des catalyseurs F et G, 10 g de ce catalyseur ont été préalablement chargés dans le réacteur puis réduits à 450 °C sous 10 litres par heures d'hydrogène. Les tests catalytiques sont ensuite effectués sous une pression absolue de 5 bars, pour un rapport molaire hydrogène sur hydrocarbure de 10 à une vitesse spatiale massique de 20 h⁻¹, entre 470 et 490 °C.

Les résultats obtenus dans ces conditions, exprimés en % poids, sont rapportés dans le tableau 4.

**Tableau 4**

| Catalyseur | température ( °C) | conversion en nC₁₀ (%pds) | rendement en oléfines (%pds) | rendement en aromatiques (%pds) |
|---|---|---|---|---|
| F | 470 | 3,2 | 2,1 | 0,3 |
| | 490 | 4,0 | 2,5 | 0,5 |
| | 470 | 2,5 | 1,7 | 0,2 |
| G | 470 | 4,2 | 2,8 | 0,6 |
| | 490 | 5,7 | 3,6 | 0,7 |
| | 470 | 3,5 | 2,5 | 0,2 |
| H | 470 | 4,2 | 2,9 | 0,2 |
| | 490 | 6,6 | 4,5 | 0,3 |
| | 470 | 4,1 | 2,9 | 0,1 |

Le catalyseur H, préparé selon l'invention directement dans le réacteur catalytique, présente des rendements en oléfines supérieurs et de plus faibles rendements en aromatiques mais surtout une meilleure stabilité,

## Revendications

1. Procédé de préparation d'un catalyseur consistant à introduire dans une masse catalytique calcinée et activée, appelée précatalyseur, et renfermant au moins un support et au moins un métal du groupe VIII de la classification périodique des éléments, au moins un métal additionnel M, caractérisé en ce que le métal additionnel est introduit sous la forme d'un composé organique pur ou dilué, in situ sous atmosphère inerte ou sous atmosphère réductrice, dans la zone de réaction où se produira le traitement d'une charge en présence dudit catalyseur ou dans une prézone de reaction directement en contact avec la zone de réaction.

2. Procédé selon la revendication 1 de préparation d'un catalyseur renfermant au moins un support, au moins un métal du groupe VIII de la classification périodique des éléments et au moins un métal additionnel M, le procédé étant caractérisé en ce que
a) dans une première étape de ladite préparation, on prépare hors site un précatalyseur renfermant au moins ledit support et au moins ledit métal du groupe VIII, le précatalyseur étant à ce stade éventuellement soumis à un séchage et étant soumis à une calcination,
b) on introduit le précatalyseur dans une zone de réaction où sera envoyée la charge à traiter par ledit catalyseur ou dans une prézone de reaction directement reliée à ladite zone de réaction,
c) on soumet alors le précatalyseur à un traitement d'activation en atmosphère neutre (gaz inerte) ou en atmosphère réductrice et,
d) dans une deuxième étape de ladite préparation on introduit ledit métal additionnel M dans le précatalyseur, par mise en contact sous atmosphère inerte ou sous atmosphère réductrice d'au moins un composé organique du métal additionnel M.

3. Procédé selon l'une des revendications 1 et 2 dans lequel le métal du groupe VIII est choisi dans le groupe constitué par le platine, le palladium, le nickel et le ruthénium.

4. Procédé selon l'une des revendications 1 à 3 dans lequel le métal additionnel M est choisi dans le groupe constitué par le germanium, l'étain, le plomb, le fer, le titane et le chrome.

5. Procédé selon l'une des revendications 1 à 4 dans lequel le support renferme au moins un oxyde réfractaire.

6. Procédé selon l'une des revendications 1 à 5 dans lequel le catalyseur renferme également au moins un métal alcalin ou alcalino-terreux.

7. Procédé selon l'une des revendications 1 à 6 dans lequel le catalyseur renferme également au moins un métalloïde.

8. Procédé selon la revendication 7 dans lequel le métalloïde est le soufre.

9. Procédé selon l'une des revendications 1 à 8 dans lequel le catalyseur renferme au moins un halogène ou un composé halogène.

10. Procédé selon l'une des revendications 1 à 9 dans lequel, au cours de ladite deuxième étape, le composé organique du métal additionnel M est introduit en phase liquide ou en phase gazeuse.

11. Procédé selon l'une des revendications 1 à 10 dans lequel, au cours de ladite deuxième étape, le composé organique du métal additionnel M est introduit sur le catalyseur dans au moins un solvant d'imprégnation lequel est une solution hydrocarbonée.

12. Procédé selon l'une des revendications 1 à 11 dans lequel l'activation du précatalyseur est réalisée en atmosphère réductrice, en présence d'hydrogène entre 300 et 600 °C.

13. Procédé selon l'une des revendications 11 et 12 dans lequel, à l'issue de la préparation du catalyseur, on procède (avant de mettre la charge à traiter au contact du catalyseur) à l'élimination du solvant d'imprégnation du métal additionnel M, puis à un traitement thermique.

14. Procédé selon la revendication 13 dans lequel ledit traitement thermique est effectué sous débit d'hydrogène, à une température comprise entre 300 et 600 °C.

15. Procédé selon l'une des revendications 11 à 14 dans lequel le métal additionnel M est introduit dans le précatalyseur sous la forme d'au moins un composé organométallique ou alcoolate.

16. Procédé selon la revendication 15 dans lequel ledit composé organométallique ou alcoolate est choisi dans le groupe formé par les complexes carbonyles ou polycétoniques du métal M et les hydrocarbymétaux du métal M tels que les alkyles, cycloalkyles, aryles, alkylaryles et arylalkyles.

17. Procédé selon l'une des revendications 11 à 16 dans lequel ledit composé organique de métal M est choisi dans le groupe constitué par l'hexacarbonyle de fer, l'isopropylate de titane, le dichlorodicyclopentadiényle de titane, le tétrabutylétain, le tétraméthylétain, le tétrapropylgermanium, le diphénylétain et le tétraéthyl plomb.

18. Procédé selon l'une des revendications 11 à 17 dans lequel ledit solvant d'imprégnation dudit métal M est choisi dans le groupe constitué par les solvants organiques oxygénés comportant de 2 à 8 atomes de carbone par molécule, les hydrocarbures paraffiniques, naphténiques et aromatiques contenant 6 à 15 atomes de carbone par molécule et les composés organiques halogénés contenant 1 à 15 atomes de carbone par molécule.

19. Procédé selon la revendication 18 dans lequel le solvant d'imprégnation est choisi dans le groupe constitué pat l'éthanol, le tétrahydrofuranne, le n-heptane, le méthylcyclohexane, le toluène et le chloroforme.

20. Procédé selon l'une des revendications 1 à 19 dans lequel au début du paragraphe c) de la revendication 1, le précatalyseur est soumis à séchage éventuel et calcination éventuelle.

21. Procédé selon l'une des revendications 1 à 20, dans lequel au cours de la fabrication, le catalyseur a été soumis à une oxychloration avant l'addition du métal additionnel M.

22. Catalyseur préparé selon le procédé décrit dans l'une des revendications 1 à 21.

23. Utilisation d'un catalyseur selon la revendication 22, ou préparé selon l'une des revendications 1 à 21 dans les réactions de déshydrogénation de paraffines.

## Claims

1. A process for the preparation of a catalyst consisting in introducing at least one additional metal M into a calcined and activated catalytic mass termed a precatalyst, the precatalyst comprising at least one support and at least one metal from group VIII of the periodic classification of the elements, characterized in that the additional metal is introduced in the form of an organic compound, pure of diluted, in situ in an inert or reducing atmosphere, into the reaction zone where a feedstock is to be treated in the presence of said catalyst or into a reaction prezone directly in contact with the reaction zone.

2. Process according to claim 1 for the preparation of a catalyst comprising at least one support, at least one metal from group VIII of the periodic classification of the elements and at least one additional metal M, the process being characterised in that
a) in a first step of said preparation, a precatalyst is prepared offsite which comprises at least one said support and at least one said group VIII metal, the precatalyst at this point being optionally dried and being calcined,
b) the precatalyst is introduced into the reaction zone where the feedstock to be treated by said catalyst will be fed, or into a reaction prezone directly connected to said reaction zone,
c) the precatalyst is then activated in a neutral atmosphere (inert gas) or a reducing atmosphere, and
d) in a second step of said preparation, said additional metal M is introduced into the precatalyst by bringing it into contact with at least one organic compound of additional metal M in an inert or reducing atmosphere.

3. Process according to claim 1 or claim 2 wherein the group VIII metal is selected from the group constituted by platinum, palladium, nickel and ruthenium.

4. Process according to any one of claims 1 to 3 wherein additional metal M is selected from the group constituted by germanium, tin, lead, iron, titanium and chromium.

5. Process according to any one of claims 1 to 4 wherein the support comprises at least one refractory oxide.

6. Process according to any one of claims 1 to 5 wherein the catalyst further contains at least one alkali or alkaline-earth metal.

7. Process according to any one of claims 1 to 6 wherein the catalyst also contains at least one metalloid.

8. Process according to claim 7 wherein the metalloid is sulphur.

9. Process according to any one of claims 1 to 8 wherein the catalyst comprises at least one halogen or halogenated compound.

10. Process according to any one of claims 1 to 9 wherein, during the second step, the organic compound of additional metal M is introduced in the liquid or gaseous phase.

11. Process according to any one of claims 1 to 9 wherein, during the second step, the organic compound of metal M is introduced into the catalyst in at least one impregnating solvent which is a hydrocarbon solution.

12. Process according to any one of claims 1 to 11 wherein the precatalyst is activated in a reducing atmosphere, in the presence of hydrogen between 300°C and 600°C.

13. Process according to any one of claims 11 and 12 wherein, when the catalyst has been prepared, the additional metal M impregnating solvent is eliminated and the catalyst is thermally treated before the feedstock is brought into contact with the catalyst.

14. Process according to claim 13 wherein said thermal treatment is carried out in a current of hydrogen, at a temperature between 300°C and 600°C.

15. Process according to any one of claims 11 to 14 wherein additional metal M is introduced into the precatalyst in the form of at least one organometallic compound or alcoholate.

16. Process according to claim 15 wherein said organometallic compound or alcoholate is selected from the group formed by carbonyl or polyketone complexes of metals M and metallic hydrocarbons of metal M such as alkyls, cycloalkyls, aryls, alkylaryls and arylalkyls.

17. Process according to any one of claims 11 to 16 wherein said organic compound of metal M is selected from the group constituted by hexacarbonyl iron, titanium isopropylate, dichlorodicyclopentadienyl titanium, tetrabutyl tin, tetramethyl tin, tetrapropyl germanium, diphenyl tin and tetraethyl lead.

18. Process according to any one of claims 11 to 17 wherein said impregnating solvent is selected from the group constituted by oxygenated organic solvents containing 2 to 8 carbon atoms per molecule, and paraffinic, naphthenic or aromatic hydrocarbons containing 6 to 15 carbon atoms per molecule and halogenated organic compounds containing 1 to 15 carbon atoms per molecule.

19. Process according to claim 18 wherein the impregnating solvent is selected from the group constituted by ethanol, tetrahydrofuran, n-heptane, methylcyclohexane, toluene and chloroform.

20. Process according to any one of claims 1 to 19 wherein, at the beginning of paragraph c) in claim 1, the precatalyst is optionally dried and optionally calcined.

21. Process according to any one of claims 1 to 20 wherein, during manufacture, the catalyst is oxychlorinated before addition of additional metal M.

22. Catalyst prepared according to a process described in any one of claims 1 to 21.

23. Use in paraffin dehydrogenation reactions of a catalyst according to claim 22 or prepared in accordance with any one of claim 1 to 21.

## Patentansprüche

1. Verfahren zur Herstellung eines Katalysators, das darin besteht, in eine calcinierte und aktivierte katalytische Masse, Vorkatalysator genannt, und die wenigstens einen Träger und wenigstens ein Metall der Gruppe VIII des Periodensystems der Elemente umfaßt, wenigstens ein Zusatzmetall M einzuführen, dadurch gekennzeichnet, daß das Zusatzmetall in Form einer organisch reinen oder verdünnten Verbindung, in situ unter inerter Atmosphäre und unter reduzierender Atmosphäre in der Reduktionszone eingeführt wird, wo die Behandlung einer Charge in Anwesenheit eines Katalysators stattfindet oder in einer Reaktionsvorzone, die direkt in Kontakt mit der Reaktionszone steht.

2. Verfahren nach Anspruch 1 zur Herstellung eines Katalysators, der wenigstens einen Träger, wenigstens ein Metall der Gruppe VIII des Periodensystem der Elemente und wenigstens ein Zusatzmetall M umfaßt, wobei das Verfahren sich dadurch auszeichnet, daß
a) in einer ersten Stufe dieser Herstellung man ex situ einen Vorkatalysator herstellt, der wenigstens diesen Träger und wenigstens dieses Metall der Gruppe VIII umfaßt, wobei der Vorkatalysator in diesem Stadium gegebenenfalls einer Trocknung ausgesetzt und einer Calcinierung unterworfen wird,
b) man den Vorkatalysator in eine Reaktionszone, wo die durch diesen Katalysator zu behandelnde Charge gegeben wird, oder in eine Reaktionsvorzone einführt, die direkt mit dieser Reaktionszone verbunden ist,
c) man dann den Vorkatalysator einer Aktivierungsbehandlung in neutraler Atmosphäre (inertes Gas) oder in reduzierender Atmosphäre aussetzt und
d) in einer zweiten Stufe dieser Herstellung man dieses Zusatzmetall M in den Vorkatalysator durch Kontaktieren unter inerter Atmosphäre oder reduzierender Atmosphäre wenigstens einer organischen Verbindung des Zusatzmetalls M einführt.

3. Verfahren nach einem der Ansprüche 1 und 2, bei dem das Metall der Gruppe VIII gewählt ist aus der Gruppe, die durch Platin, Palladium, Nickel und Ruthenium gebildet ist.

4. Verfahren nach einem der Ansprüche 1 bis 3, wobei das Zusatzmetall M gewählt wird aus der Gruppe, die durch Germanium, Zinn, Blei, Eisen, Titan und Chrom gebildet ist.

5. Verfahren nach einem der Ansprüche 1 bis 4, bei dem der Träger wenigstens ein feuerfestes Oxyd umfaßt.

6. Verfahren nach einem der Ansprüche 1 bis 5, bei dem der Katalysator auch wenigstens ein Alkali oder Erdalkalimetall einschließt.

7. Verfahren nach einem der Ansprüche 1 bis 6, bei dem der Katalysator auch wenigstens ein Metalloid einschließt.

8. Verfahren nach Anspruch 7, bei dem das Metalloid Schwefel ist.

9. Verfahren nach einem der Ansprüche 1 bis 8, bei dem der Katalysator wenigstens ein Halogen oder eine Halogenverbindung einschließt.

10. Verfahren nach einem der Ansprüche 1 bis 9, bei dem wenigstens während dieser zweiten Stufe die organische Verbindung des Zusatzmetalls M in flüssiger Phase oder in gasförmiger Phase eingeführt wird.

11. Verfahren nach einem der Ansprüche 1 bis 10, bei dem während dieser zweiten Stufe die organische Verbindung des Zusatzmetalls M auf den Katalysator in wenigstens einem Imprägnierungslösemittel gegeben wird, bei dem es sich um eine kohlenwasserstoffhaltige Lösung handelt.

12. Verfahren nach einem der Ansprüche 1 bis 10, bei dem die Aktivierung des Vorkatalysators in reduzierender Atmosphäre in Anwesenheit von Wasserstoff zwischen 300 und 600°C durchgeführt wird.

13. Verfahren nach einem der Ansprüche 11 und 12, bei dem bei Beendigung der Herstellung des Katalysators man (bevor die zu behandelnde Charge in Kontakt mit dem Katalysator gebracht wird) die Eliminierung des Imprägnierungslösungsmittels des Zusatzmetalls M, dann eine thermische Behandlung vorgenommen wird.

14. Verfahren nach Anspruch 13, bei dem diese thermische Behandlung unter einem Wasserstoffstrom bei einer Temperatur zwischen 300 und 600°C vorgenommen wird.

15. Verfahren nach einem der Ansprüche 11 bis 14, bei dem das Zusatzmetall M in den Vorkatalysator in Form wenigstens einer organometallischen Verbindung oder eines Alkoholats eingeführt wird.

16. Verfahren nach Anspruch 15, bei dem diese organometallische Verbindung oder das Alkoholat gewählt ist aus der Gruppe, die gebildet wird durch die Carbonyl- oder Polyketonkomplexe des Metalls M sowie die Hydrocarbylmetalle des Metalls M wie die Alkyle, Cycloalkyle, Aryle, Alkylaryle und Arylalkyle.

17. Verfahren nach einem der Ansprüche 11 bis 16, bei dem diese organische Verbindung des Metalls M gewählt ist aus der Gruppe, die gebildet wird durch Eisenhexacarbonyl, Titanisopropylat, Titandichlordicyclopentadienyl, Zinntetrabutyl, Zinntetramethyl, Germaniumtetrapropyl, Zinndiphenyl und Bleitetraethyl.

18. Verfahren nach einem der Ansprüche 11 bis 17, bei dem dieses Imprägnierungslösungsmittel für dieses Metall M gewählt ist aus der Gruppe, die gebildet wird durch die organischen sauerstoffhaltigen Lösungsmittel, welche 2 bis 8 Kohlenstoffatome pro Molekül enthalten, die paraffinischen, naphtenischen und aromatischen Kohlenwasserstoffe, die 6 bis 15 Kohlenstoffatome pro Molekül enthalten sowie die haloginierten organischen Verbindungen, die eins bis fünf Kohlenstoffatome pro Molekül enthalten.

19. Verfahren nach Anspruch 18, bei dem das Imprägnierungslösungsmittel gewählt ist aus der Gruppe, die gebildet wird durch Ethanol, Tetrahydrofuran, n-Heptan, Methylcyclohexan, Toluol und Chloroform.

20. Verfahren nach einem der Ansprüche 1 bis 19, bei dem zu Beginn des Absatzes c) des Anspruchs 1, der Vorkatalysator einer eventuellen Trocknung und einer eventuellen Calcinierung ausgesetzt wird.

21. Verfahren nach einem der Ansprüche 1 bis 20, bei dem während der Herstellung der Katalysator einer Oxichlorierung vor der Zugabe des Zusatzes mit Zusatzmetall M ausgesetzt wurde.

22. Katalysator, hergestellt nach den in den Ansprüchen 1 bis 21 beschriebenen Verfahren.

23. Verwendung eines Katalysators nach Anspruch 22 oder hergestellt nach einem der Ansprüche 1 bis 21 in den Dehydrierungsreaktionen von Paraffinen.
